(19) Europäisches Patentamt / European Patent Office / Office européen des brevets

(11) **EP 3 084 426 B1**

(12) **EUROPEAN PATENT SPECIFICATION**

(45) Date of publication and mention
of the grant of the patent:
**15.04.2020  Bulletin 2020/16**

(21) Application number: **14871681.4**

(22) Date of filing: **10.12.2014**

(51) Int Cl.:
$G16B\ 30/20$ *(2019.01)*     $G16B\ 40/20$ *(2019.01)*

(86) International application number:
**PCT/US2014/069539**

(87) International publication number:
**WO 2015/094854 (25.06.2015 Gazette 2015/25)**

(54) **ITERATIVE CLUSTERING OF SEQUENCE READS FOR ERROR CORRECTION**

ITERATIVES CLUSTERN VON SEQUENZABLESUNGEN ZUR FEHLERKORREKTUR

REGROUPEMENT ITÉRATIF DE LECTURES DE SÉQUENCES POUR CORRECTION D'ERREUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2013  US 201361917777 P
24.07.2014  US 201462028741 P**

(43) Date of publication of application:
**26.10.2016  Bulletin 2016/43**

(73) Proprietor: **Pacific Biosciences Of California, Inc.
Menlo Park, CA 94025 (US)**

(72) Inventor: **TSENG, Huei-Hun
Menlo Park, CA 94025 (US)**

(74) Representative: **Mewburn Ellis LLP
City Tower
40 Basinghall Street
London EC2V 5DE (GB)**

(56) References cited:
WO-A1-2009/091798     WO-A1-2013/181170
US-A1- 2004 171 051     US-A1- 2012 330 566
US-A1- 2013 137 588     US-A1- 2013 196 321

• Carl Meyer ET AL: "Determining the Number of Clusters via Iterative Consensus Clustering" In: "Proceedings of the 2013 SIAM International Conference on Data Mining", 2 May 2013 (2013-05-02), Society for Industrial and Applied Mathematics, Philadelphia, PA, XP055388637, ISBN: 978-1-61197-283-2 pages 94-102, DOI: 10.1137/1.9781611972832.11, * the whole document *
• Elizabeth Tseng ET AL: "FIND MEANING IN COMPLEXITY Iso-Seq TM Bioinformatics Analysis of the Human MCF-7 Transcriptome Sequenced with PacBio Long Reads", , 31 December 2004 (2004-12-31), XP055388740, Retrieved from the Internet: URL:https://s3.amazonaws.com/files.pacb.com/pdf/Iso-Seq+Bioinformatics+Analysis+of+the+Human+MCF-7+Transcriptome.pdf[retrieved on 2017-07-06]

**Description**

BACKGROUND OF THE INVENTION

Advances in biomolecule sequence determination, in particular with respect to nucleic acid and protein samples, has revolutionized the fields of cellular and molecular biology. Facilitated by the development of automated sequencing systems, it is now possible to sequence mixed populations of sample nucleic acids. However, the quality of the sequence information must be carefully monitored, and may be compromised by many factors related to the biomolecule itself or the sequencing system used, including the composition of the biomolecule (e.g., base composition of a nucleic acid molecule), experimental and systematic noise, variations in observed signal strength, and differences in reaction efficiencies. As such, processes must be implemented to analyze and improve the quality of the data from such sequencing technologies.

Besides affecting overall accuracy of sequence reads generated, these factors can complicate designation of a base-call as a true variant or, alternatively, a miscall (e.g., insertion, deletion, or mismatch error in the sequence read). For example, when sequence reads have the base calls that differ between the homologous chromosomes, it is important to be able to determine whether the differing base calls are true variations between the homologues, or are merely sequencing errors. Yet further, a viral population in an individual can have many variations between individual viral genomes in the population, especially in highly mutable viruses such as HIV. Being able to identify different sequencing reads that have different origins (e.g., different chromosome or genome origins) is key to being able to accurately characterize a mixed population of nucleic acids. For a theoretical sequencing platform that generates reads that are 100% accurate, the reads can simply be compared to one another with simple string matching algorithms. Any difference between the reads is indicative of a true variant, and therefore, a different origin. However, any real-world raw sequencing data is likely to contain errors, so a simple string matching algorithmic approach will not be sufficient. This is especially true when sequencing transcriptomes.

A transcriptome is a set of all RNA molecules, including mRNA, rRNA, tRNA, and other non-coding RNAs produced in one or a population of cells. Because the term includes all mRNA transcripts in the cell, the transcriptome reflects the genes that are being actively expressed at any given time. Currently, there are two general methods of inferring transcriptomes. One approach maps sequence reads onto a reference genome, either of the organism whose transcriptome is being studied or of a closely related species. The other approach is de novo transcriptome assembly that uses software to infer transcripts directly from short sequence reads.

However, commercially available genome aligners do not enable error correction of full-length long sequence reads in transcriptome sequencing. For example, reads produced on the PacBio® RS II instrument average about 5-6 kb, and reads as long as 20 kb are routinely generated. With such long-read capabilities, full-length mRNA transcripts can be sequenced, e.g., after conversion to cDNA. This can help the researcher identify splicing patterns that are difficult to reconstruct using short-read sequencing technologies. However, publicly available sequence aligners, e.g., GMAP, and functional annotation tools almost all require reads having near 100% accuracy. The PacBio instrument generates reads from single template molecules that have an error profile that makes it difficult to directly apply these sequence alignment tools. However, in cases where the sequencing insert (transcript) is much shorter than the polymerase readlength, highly accurate consensus sequences can be generated: through redundant sequencing of single molecules, the long length of the cDNA templates combined with the processivity of the polymerase sequencing engine in the system can result in sufficient redundancy to achieve the required accuracy for these analysis tools. This however, is only applicable to shorter transcripts, and longer transcripts would still require additional processing before they achieve the level of accuracy amenable for biological analysis.

Currently, there are two published tools for error correcting long reads (e.g., PacBio® cDNA long reads) in transcriptome sequencing, PacBioToCA and LSC. Both tools use short reads (e.g., Illumina® short reads), and follow the general schema of: for each long read, align short reads to the long read as if it were a genomic "scaffold", and create the best consensus based on the short read alignment. This general schema has several disadvantages: (1) Since short reads are only 50-100 bp, they can map nonspecifically and introduce more errors; (2) All currently available short read technologies carry their own systematic errors that could bias the correction; (3) No advantage is taken of the fact that the same transcript is often represented by multiple long reads, which, in the case of long reads from Pacific Biosciences, do not have systematic biases; (4) Quality Values (QVs) from the long reads are not used; and (5) The schema requires two different sequencing systems.

What is needed is an algorithm that addresses the problem of errors in transcriptome sequencing, and preferably an algorithm designed to deal with the transcriptome de novo, i.e., without a reference genome.

BRIEF SUMMARY OF THE INVENTION

The exemplary embodiments are generally directed to processes for analyzing sequence data from mixed populations of nucleic acids, for assigning each sequence read to a particular origin, and for ultimately identifying one or more consensus sequences of one or more biomolecular target sequences from the sequence information. The methods provided herein are applicable not only to sequence data having few errors, but also to sequence data having relatively high rates of insertions, deletions, and/or mismatch errors. Consequently, the invention is also directed to systems that carry out these processes.

The invention and various specific aspects and embodiments will be better understood with reference to the following detailed descriptions and figures, in which the invention is described in terms of various specific aspects and embodiments. These are provided for purposes of clarity and should not be taken to limit the invention.

Accordingly, the present invention provides a method for iterative clustering of sequence reads for error correction, the method performed by at least one software component executing on at least one processor, comprising:

(A) receiving a set of sequence reads and associated quality values, wherein the associated quality values are estimations of per-position base call accuracy;
(B) grouping the sequence reads into a set of initial clusters based on sequence similarity;
(C) generating a cluster consensus for each of the initial clusters;
(D) iteratively improving the clustering based on the cluster consensus and the quality values associated with the sequence reads, wherein iteratively improving the clustering comprises:

(i) calculating a probability of each sequence read belonging to each cluster using the quality values,
(ii) reassigning individual sequence reads from one cluster to another cluster having a highest calculated probability, and
(iii) merging highly similar clusters; and

(E) generating and outputting a final cluster consensus for each of the clusters.

In one embodiment, the input sequence reads comprise full-length long reads of at least .5 kb in length through 1, 2, 3, 4, 5, 7, or 10 kb in length, and the final cluster consensus is generated using the cluster consensus and non-full-length reads, which are used to impart full coverage to the sequence data to provide a higher level of consensus.

BRIEF DESCRIPTION OF SEVERAL VIEWS OF THE DRAWINGS

Figure 1 is a diagram illustrating one embodiment of a computer system for implementing a process for using iterative clustering of sequencing reads for error correction of transcriptome sequencing data.
Figure 2 is a flow diagram illustrating certain aspects of a process for iterative clustering of sequence reads for error correcting by according to an exemplary embodiment.
Figure 3 is a diagram showing an example portion of two reads from the same isoform that have been aligned to create a pairwise alignment.
Figure 4 is a diagram illustrating exemplary similarity graphs.
Figure 5 is a diagram illustrating one implementation for distinguishing a true isoform difference from sequence errors between aligned reads.
Figure 6 is a diagram illustrating an example of sequence reads initially assigned to incorrect clusters, where sequence reads of the same fill pattern are from the same isoform.
Figure 7 is a diagram illustrating exemplary cluster consensus, C1, C2, C3 and C4, generated for each of clusters, respectively.
Figure 8 is a diagram illustrating reassignment of a sequence read from one cluster to the cluster having the highest computed probability of membership.
Figure 9 is a diagram illustrating an example of creating a new cluster from an orphan.
Figure 10 is a diagram illustrating the merger of two clusters.

DETAILED DESCRIPTION OF THE INVENTION

Various embodiments and components of the present invention employ signal and data analysis techniques that are familiar in a number of technical fields. For clarity of description, details of known analysis techniques are not provided herein. These techniques are discussed in a number of available reference works, such as: R. B. Ash. Real Analysis and Probability. Academic Press, New York, 1972; D. T. Bertsekas and J. N. Tsitsiklis. Introduction to Probability. 2002; K. L. Chung. Markov Chains with Stationary Transition Probabilities, 1967; W. B. Davenport and W. L Root. An Introduction to the Theory of Random Signals and Noise. McGraw-Hill, New York, 1958; S. M. Kay, Fundamentals of Statistical Processing, Vols. 1-2, (Hardcover - 1998); Monsoon H. Hayes, Statistical Digital Signal Processing and Modeling, 1996; Introduction to Statistical Signal Processing by R.M. Gray and L.D. Davisson; Modern Spectral Estimation: Theory and Application/Book and Disk (Prentice-Hall Signal Processing Series) by Steven M. Kay (Hardcover - Jan 1988); Modern Spectral Estimation: Theory and Application by Steven M. Kay (Paperback - Mar 1999); Spectral Analysis and Filter Theory in Applied Geophysics by Burkhard Buttkus (Hardcover - May 11, 2000); Spectral Analysis for Physical Applications by Donald B. Percival and Andrew T. Walden (Paperback - Jun 25, 1993); Astronomical Image and Data Analysis (Astronomy and Astrophysics Library) by J. L. Starck and F. Murtagh (Hardcover - Sep 25, 2006); Spectral Techniques In Proteomics by Daniel S. Sem (Hardcover - Mar 30, 2007); Exploration and Analysis of DNA Microarray and Protein Array Data (Wiley Series in Probability and Statistics) by Dhammika Amaratunga and Javier Cabrera (Hardcover - Oct 21, 2003).

Error correction of long reads for transcriptome analysis is different from error correction for genome assembly. Both can be formulated as a clustering problem. In genome assembly, there are only as many "clusters" as there are chromosomes; each chromosome is largely different from each other. In comparison to the whole chromosome size, the shared repeat regions are very small, and as long as there is a continuous long read spanning the repeat, it is relatively easy to resolve its origin.

In contrast, for transcriptome analysis there are as many clusters as there are transcripts. In eukaryotes, genes can have many different splice forms. In an extreme example, one isoform of a transcript has an extra 20 bp exon and the other isoform does not. For many biological problems, it is important to be able to tell the two isoforms apart. This level of detailed difference is rarely found on genomic scales, so currently available methods, e.g., Hierarchical Genome Assembly Process (HGAP), which generates high quality (>99.999% accurate) de novo assemblies cannot be directly applied to the transcriptome problem (HGAP is described in U.S. Patent Application 13/941,442, filed July 12, 2013).

The "quasispecies problem" is a specific application of the general transcriptome clustering problem. Like transcriptome sequencing, the total number of clusters is unknown and one must iteratively "guess" both the clusters and the cluster consensus. The problem is easier with respect to the HIV genome, because the HIV genome is known and priors can be placed on the number of mutations expected. Further information on the quasispecies problem is provided in Zogardi, et al. (2010) "Error correction of next-generation sequencing data and reliable estimation of HIV quasispecies," Nucl. Acids. Res. doi: 10.1093/nar/gkq655.

According to the exemplary embodiments, an algorithm is provided that addresses the problem of errors in transcriptome sequencing. However, instead of the HGAP concept of using a "seed read" for aligning shorter reads in order to generate highly accurate pre-assembled reads, the algorithm of the exemplary embodiments utilizes cluster consensus.

The exemplary embodiments relate generally to generating consensus sequences from mixed populations. More specifically, the exemplary embodiments provide methods and systems for error correction of reads based on iterative clustering of isoforms using primarily long read data. The probability of each input sequence read belonging to each cluster is iteratively calculated and the sequences are then reassigned to clusters having a higher probability of membership. In addition, the process merges highly similar clusters. According to the exemplary embodiments, iterative isoform-level clustering removes transcript redundancy and improves transcriptome consensus accuracy, all without requiring a reference genome.

Computer Implementation

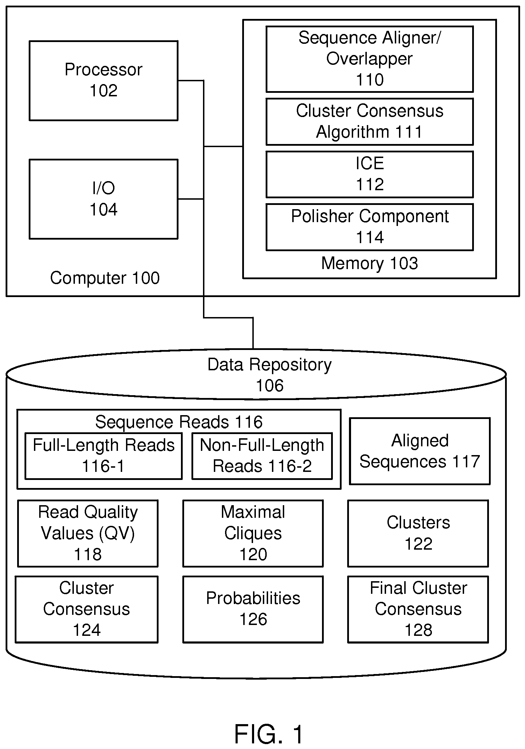

Figure 1 is a diagram illustrating one embodiment of a computer system for implementing a process for iterative clustering of sequence reads for error correction. In specific embodiments, the invention may be embodied in whole or in part as software recorded on fixed media. The computer 100 may be any electronic device having at least one processor 102 (e.g., CPU and the like), a memory 103, input/output (I/O) 104, and a data repository 106. The CPU 102, the memory 103, the I/O 104 and the data repository 106 may be connected via a system bus or buses, or alternatively using any type of communication connection. Although not shown, the computer 100 may also include a network interface for wired and/or wireless communication. In one embodiment, computer 100 may comprise a personal computer (e.g., desktop, laptop, tablet etc.), a server, a client computer, or wearable device. In another embodiment the computer 100 may comprise any type of information appliance for interacting with a remote data application, and could include such devices as an internet-enabled television, cell phone, and the like.

The processor 102 controls operation of the computer 100 and may read information (e.g., instructions and/or data) from the memory 103 and/or a data repository 106 and execute the instructions accordingly to implement the exemplary embodiments. The term "processor 102" is intended to include one processor, multiple processors, or one or more processors with multiple cores.

The I/O 104 may include any type of input devices such as a keyboard, a mouse, a microphone, etc., and any type of output devices such as a monitor and a printer, for example. In an embodiment where the computer 100 comprises a server, the output devices may be coupled to a local client computer.

The memory 103 may comprise any type of static or dynamic memory, including flash memory, DRAM, SRAM, and the like. The memory 103 may store programs and data including a sequence aligner/overlapper 110, a cluster consensus algorithm 111, an Iterative Cluster Error correction (ICE) component 112, and a polisher component 114 (e.g., Quiver). These components/algorithms may be used in the process of transcriptome sequence assembly as described herein.

The data repository 106 may store several databases including one or more databases that store sequence reads 116, read quality values (hereinafter QVs) 118, maximal cliques 120, clusters 122, cluster consensus 124, probabilities 126, and final consensus sequences 128. In the transcriptome sequencing embodiment, the sequence reads 116 comprise isoform sequence reads, which may include both full-length sequence reads (hereinafter, "full-length reads") 116-1 and non-full-length sequence reads (hereinafter, "Non-full-length reads") 116-2. Also in this embodiment, the clusters 122 may comprise isoform-level clusters.

In one embodiment, the data repository 106 may reside within the computer 100. In another embodiment, the data repository 106 may be connected to the computer 100 via a network port or external drive. The data repository 106 may comprise a separate server or any type of memory storage device (e.g., a disk-type optical or magnetic media, solid state dynamic or static memory, and the like). The data repository 106 may optionally comprise multiple auxiliary memory devices, e.g., for separate storage of input sequences (e.g., sequence reads), sequence information, calculation results and/or other information. Computer 100 can thereafter use that information to direct server or client logic, as understood in the art, to embody aspects of the invention.

In operation, an operator may interact with the computer 100 via a user interface presented on a display screen (not shown) to specify the sequence reads 116 and other parameters required by the various software programs. Once invoked, the programs in the memory 103 including the sequence aligner/overlapper 110, the cluster consensus algorithm 111, the ICE component 112, and the polisher component 114, are executed by the processor 102 to implement the methods of the present invention.

The sequence aligner/overlapper 110 reads the selected sequence reads 116 from the data repository 106 and performs sequence alignment on the sequence reads 116 to identify regions of similarity that may be a consequence of structural or functional or other relationships between the sequence reads 116. In one embodiment, the full-length reads 116-1 are generally high accuracy reads, e.g., at least about 98% or 99% accurate, and may be raw reads from a sequencing technology that provides such high quality reads, or may be pre-assembled, high-quality reads constructed from sequencing read data of a lower quality, as described elsewhere herein. Aligned sequences 117 are generated by the sequence aligner/overlaper 110 during the sequence alignment. In certain embodiments, the sequence aligner/overlaper 110 is implemented in C, C++, Java, C#, F#, Python, Perl, Haskell, Scala, Lisp, a Python/C hybrid, and others known in the art.

The ICE component 112 generates clusters 122 of similar sequence reads by grouping the sequence reads 116 into a set of initial clusters based on similarity and maximal cliques 120. The cluster consensus algorithm 111 generates the cluster consensus 124 for each of the clusters. The ICE component 112 then iteratively improves the clustering through iterations of based on the cluster consensus 124 and the quality values associated with the sequence reads, which includes reassigning sequence reads 116 from one cluster to another based on computer probabilities 126, and merging substantially similar clusters. The polisher component 114 may then generate the final cluster consensus 128 for each of the clusters 122 in accordance with exemplary embodiments, as explained further below.

The output of the processing may include is a list of final consensus sequences 128, each of which represents the "consensus" of a cluster. In one embodiment, each of the clusters 122 may represent a single, unique transcript. Thus, in one embodiment, the present invention may provide methods and systems for identifying a set of unique full-length transcripts from a mixed population using full-length reads 116-1.

In one embodiment, the results of the process may optionally further comprise quality information, technology information (e.g., peak characteristics, expected error rates), alternate (e.g., second or third best) consensus determination, confidence metrics, and the like. During and after the process of creating the initial clusters, generating cluster consensus, iterative clustering, and generation of the final cluster consensus, the progress and/or result of this processing may be saved to the memory 103 and the data repository 106 and/or output through the I/O 104 for display on a display device and/or saved to an additional storage device (e.g., CD, DVD, Blu-ray, flash memory card, etc.), or printed.

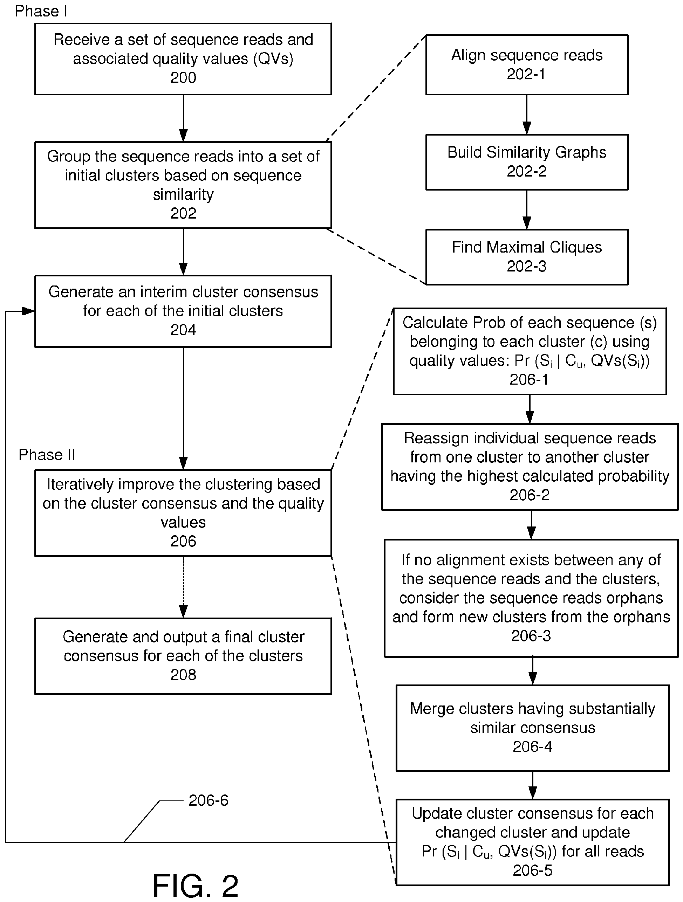

Figure 2 is a flow diagram illustrating certain aspects of a process for iterative clustering of sequence reads for error correcting by according to an exemplary embodiment. In one embodiment, the process may be used to correct errors in long reads during transcriptome sequencing. The process may be performed by a combination of the sequence aligner/overlapper 110, the cluster consensus algorithm 111, the ICE component 112, and the polisher component 114 (Figure 1), which although are shown as separate components, the functionality of each may be combined into a lesser or greater number of software algorithms/components.

The process may begin by receiving a set of sequence reads 116 and associated quality values 118 (block 200). The sequence reads 116 preferably comprise, but are not limited to, a set full-length long reads 116-1. The quality values (QVs) 118 are estimations of per-position base call accuracy generated by a sequencing machine.

The iterative clustering error correction (ICE) component 112 groups the sequence reads into a set of initial clusters based on sequence similarity (block 202). The cluster consensus algorithm 111 generates the cluster consensus 124 for each of the initial clusters (block 204). The ICE component 112 iteratively improves clustering based on the cluster consensus and the quality values 118 associated with the sequence reads (block 206), as described further below.

In further embodiments, the process further includes generating and outputting a final cluster consensus 128 for each of the clusters of (block 208). In one embodiment, the final cluster consensus 128 may comprise a list of final cluster consensus sequences, each of which represents the consensus sequence of a cluster (and therefore a transcript in one embodiment). In one embodiment, the final cluster consensus 128 may be generated once the iterative clustering process has completed. In another embodiment where the input comprises full-length reads 116-1, the final cluster consensus may be generated by inputting non-full-length reads 116-2 into a final polishing process, which then generates the final cluster consensus 128. As is well known in the art, the final cluster consensus 128 may be saved e.g. in the memory 103 and/or the data repository 106, or sent to the I/O 104 for display on a monitor and/printing by a printer.

The above steps are described in further detail below.

Sequence Reads

One goal of transcript isoform sequencing is to understand transcriptome complexity using accurate, unassembled, full-length long reads. The full-length reads 116-1 are captured and identified automatically by a sequencing machine, but the exemplary embodiments improve the accuracy through iterative clustering.

In the exemplary embodiment, the input sequence reads 116 comprise the full-length long reads 116-1 of a transcript, for example. However in another embodiment, the input sequence reads 116 may comprise the non-full-length reads 116-2. The sequence reads 116 can optionally comprise redundant sequence information, e.g., where the same transcript was repeatedly sequenced to generate a long sequence read comprising multiple copies of the transcript. Further, the additional information associated with the sequence reads 116 may comprise features of underlying sequencing technology output (e.g., trace characteristics (integrated counts per peak, shape/height/width of peaks, distance to neighboring peaks, characteristics of neighboring peaks), signal-to-noise ratios, power-to-noise ratio, background metrics, signal strength, reaction kinetics, etc.), and the like.

Initial Clustering

The iterative clustering process comprises two phases. The first phase includes grouping the sequence reads 116 into a set of initial clusters based on sequence similarity (block 202). In one embodiment, for example, initial clustering is used to help determine which sequence reads 116 originate from the same transcript isoform. The background idea for clustering is the observation of multiple sequence reads originating from multiple copies of the same isoform. For example, the following shows three copies of a transcript read originating the same isoform:

T**G**GGAGC**C**TATGCGACAAT**G**AAACCTG...
TGGAGC**A**ATATGCGAACAATAAAACCT**C**...
TGGAGCATATGCGAACAATAAAAC**GG**G...

where the bolded bases represent randomly distributed errors that are primarily indels (insertions or deletions). Clustering such reads originating from the same isoform may lead to higher accuracy consensus sequences.

Alignment

Figure 2 shows further details of the initial clustering process (block 202). In one embodiment, the initial clustering process may begin by the sequence aligner/overlapper 110 aligning the sequence reads 116 to create aligned reads. Many known sequence alignment processes may be used, such as for example, mapping single molecule sequencing reads 116 using a Basic Local Alignment with Successive Refinement (BLASR) algorithm, further described in U.S. Patent Publication No. 20120330566.

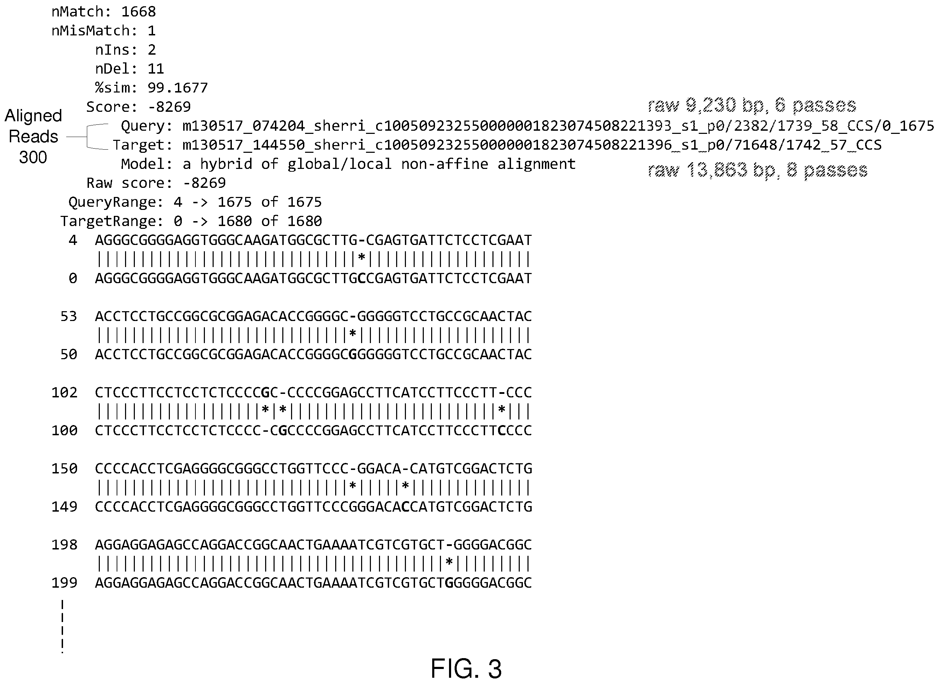

Figure 3 is a diagram showing an example portion of two reads from the same isoform that have been aligned using the sequence aligner/overlapper 110, creating aligned reads 300. In this example, the first aligned read shown as "Query" has a length of 1,675 bp, and the second aligned read shown as "Target" has a length of 1,680 bp. The alignment ("nMatch") between the aligned reads 300 is 1.6 kbp and the percent similarity ("%sim") is 99.1677, which includes 2 insertions and 11 deletion indels (indicated by "*").

After alignment, the next step is to form isoform clusters. One could use a reference genome and align the reads to the reference genome and determine that the reads at a particular locus represent an isoform. However, there are many alternative isoforms per locus that would remain unresolved. In addition, this approach relies on an aligner and requires a good reference genome to begin with, limiting the approach to those applications having a pre-existing reference genome.

According to one exemplary embodiment, methods and systems are provided for identifying isoform clusters that do not use a reference genome, and are therefore suitable for use in applications in which no reference genome exists.

Similarity Graph

Referring again to Figure 2, after alignment, the aligned reads 300 are used to build a similarity graph (block 202-2). The similarity graph is constructed such that each sequence read 116 is represented as a node in the graph, and alignments between the sequence reads 116 are represented as connecting edges between the nodes to indicate that the two sequence reads have an alignment hit (i.e., a sufficiently high percent of similarity).

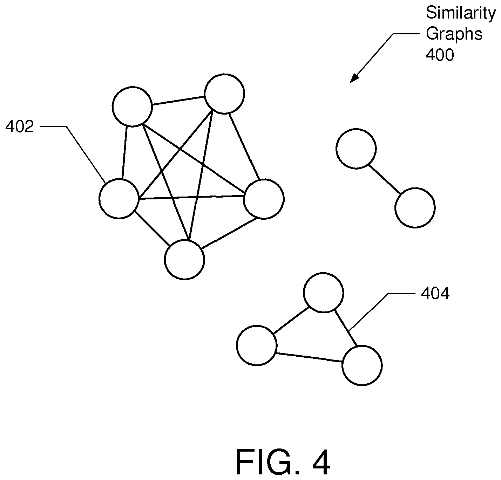

Figure 4 is a diagram illustrating exemplary similarity graphs 400. The algorithm for finding isoform clusters utilizes pairwise alignment in which each node 402 in a similarity graph 400 represents a read, and edges 404 connecting pairs of nodes represent the presence of pairwise alignment, such as that shown in Figure 3 where the query and target reads would be represented in the graph by a pair of nodes 402 and connected by an edge 404 due to their high percentage of similarity.

Maximal Cliques

Typically, the similarity graph process results in multiple similarity graphs 400 being formed. Referring again to Figure 2, this is followed by finding all maximal cliques in the similarity graphs (block 202-3). A clique refers to a graph comprising a set of nodes in which for every two nodes 402, there exists an edge connecting the two. A maximal clique is a clique of the largest possible size that does not contain a node 402 that overlaps with another clique. The maximal clique finding algorithm non-deterministically partitions the similarity graphs 400 into non-overlapping maximal cliques. There are many approaches to finding all maximal cliques. In one embodiment, a maximal clique finding algorithm may be run, such as a greedy randomized adaptive search procedure that iteratively constructs a randomized, greedy biased solution, which is then expanded to a local optimal solution. See for example, Abello et al., On maximum clique problems in very large graphs, AT&T labs Research Technical Report, 1998.

Partitioning the similarity graphs 400 into non-overlapping maximal cliques requires comparing the sequence reads 116 to detect isoform alignment differences to determine if the sequence reads 116 belong in the same clique. One method for detecting isoform alignment differences is to detect large gaps in alignment between two aligned reads. For example, if given two aligned reads, where one has a large insertion with respect to the other one, then it is very likely the insertion is an extra xenon, and therefore, an isoform difference can been detected. However, detecting isoform alignment differences becomes more problematic as the gaps in alignment become smaller and smaller. For example, only a seven base insertion difference between two aligned reads may indicate a polymer stretch. What needs to be determined is whether this is a true isoform difference or sequence error.

According to one aspect of the exemplary embodiment, determining an isoform difference from a sequence error may be made by leveraging the fact that every base from the raw read sequences 116, including insertions, is associated with a quality values (QV) that estimates per-position accuracy and indicates the probability of whether each base is a substitution error, an insertion error, or deletion error.

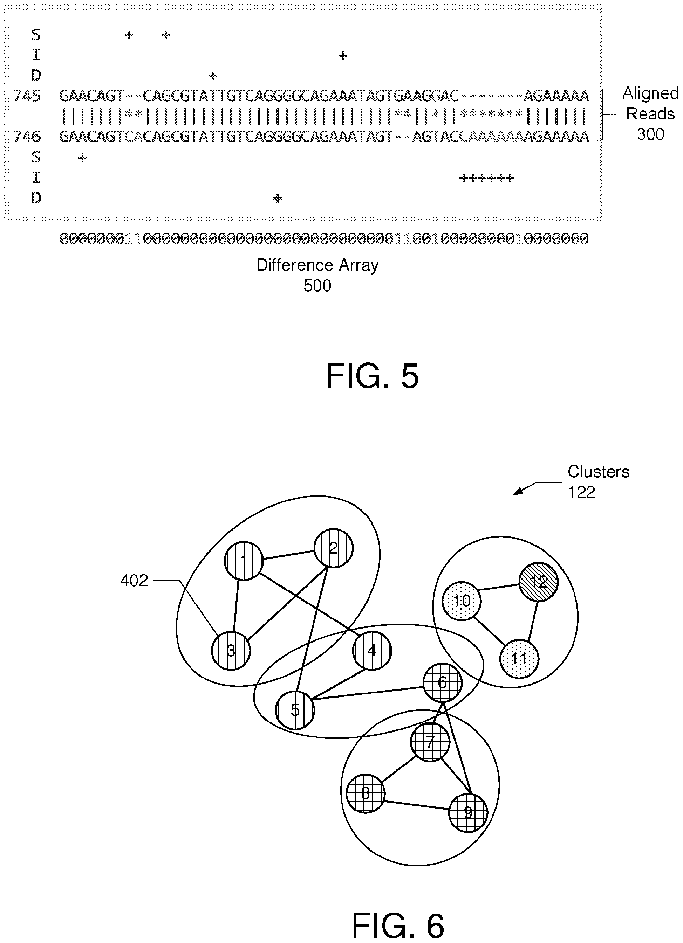

Figure 5 is a diagram illustrating one implementation for distinguishing an isoform difference from sequence errors between aligned reads 300. In one embodiment, a difference array 500 may be used to keep track of positional differences between the two aligned reads 300. Rows for substitutions (S), insertions (I), and deletions (D) above and below the two aligned reads 300 having a "+"at each base position show where the associated QVs 118 indicate that it is sufficiently likely an error occurred. Each position in the difference array 500 may include a value, e.g., 0 or 1, where a 0 value indicates the differences between the two aligned reads 300 is caused by a sequencing error (rather than a true isoform difference), and a 1 value indicates the differences between the two aligned reads 300 cannot be explained by sequencing errors.

It is then determined if there is any sufficiently large regions of 1 values in the difference array, i.e., looking for a region in the difference array from [I, J] having a range greater than or equal to a threshold length T, and the sum of the region of 1 values is greater than a percentage threshold C:

$J - I \geq T$ and $\mathrm{sum}(D[I{:}J]) \geq C * T$

For example, assume that the threshold length T is set to 10 bases, and the percentage threshold C is set to 50%. The difference array 500 would be searched for a region longer than 10 bases in which more than 50% of the bases have a value of 1. If no such region can be found, then the two aligned reads 300 may be considered to be from the same isoform. In the example shown in Figure 5, no such a region exists in the difference array 500 so the two aligned reads 300 are determined to be from the same isoform, and hence placed in the same clique. If, on the other hand, such a region is found, the two aligned reads 300 would be determined to be from different isoforms and hence not placed the same clique. For further information see Tseng & Tompa, Algorithms for Locating Extremely Conserved Elements in Multiple Sequence Alignments, BMC Bioinformatics (2009).

Note that a clique by definition requires each node 402 to be interconnected. According to the exemplary embodiments, after the maximal clique finding process, the term "clique" will be replaced below with the broader term "cluster" because the edges 404 between the nodes are not required or used after the maximal clique finding process.

After the alignment, building the similarity graphs and maximal clique finding processes (blocks 202-1 through 202-3), problems with how the sequence reads 116 are grouped may still typically remain. That is, there may be ambiguity in the first set of clusters that are formed. For example, in Figure 4, the maximal clique finding process may find ambiguity with respect to a pair of nodes/reads 402 as to which clique the nodes/reads 402 belong. Maximal clique finding is only an initial estimate of the membership of each clique. Therefore, at the end of phase 1 of the process, some sequence reads 116 may be assigned to incorrect clusters, and some sequence reads 116 that should belong together may be assigned to separate clusters.

Figure 6 is a diagram illustrating an example of sequence reads initially assigned to incorrect clusters 122, where sequence reads/nodes of the same fill pattern are from the same isoform. As shown, sequence reads labeled 1-3 have been incorrectly placed in a different cluster than sequence reads 4-5, all of which are from the same isoform. In addition, sequence reads 11 and 12 are incorrectly grouped with read 12; and read 6 has been incorrectly separately grouped from reads 7-9.

Referring again to Figure 2, according to the exemplary embodiments, the processes performed after the initial clustering 202 are designed to resolve the ambiguities of the initial clusters 122.

Cluster Consensus

After the initial clusters 122 are formed, the cluster consensus algorithm 111 generates the cluster consensus 124 for each of the initial clusters (block 204), where each cluster consensus 124 is used to represent the sequence of all the members of the cluster. Cluster consensus generation is well-known in the art. For example, the cluster consensus algorithm 111 may be based on using directed acyclic graphs to encode multiple sequence alignment, e.g., the DAGCon (Directed Acyclic Graph Consensus) algorithm. Given a collection of aligned reads 300, DAGCon takes a group of paired alignments that are aligned against a reference or backbone/seed to which other reads are aligned (in de novo genome assembly, the longest sequence reads are used as the backbone/seed) to generate a directed acyclic graph, where each path through the graph represents one of the alignments. The graph is then simplified and a most likely path through the graph is determined, which is the consensus. See Chin et al., Nonhybrid, finished microbial genome assemblies from long-read SMRT sequencing data, Nature Methods (2013).

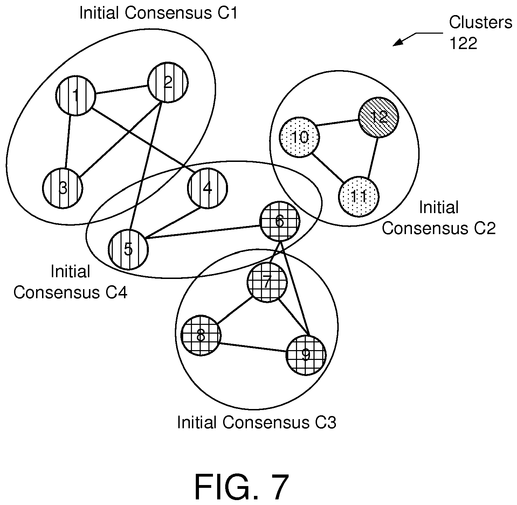

Figure 7 is a diagram illustrating exemplary cluster consensus, C1, C2, C3 and C4, generated for each of clusters 122, respectively, where each of the input read sequences 116 belongs to exactly one cluster 122$_{[DA1]}$.

Referring again to Figure 2, after the cluster consensus generation (block 204), the second phase of the iterative clustering for error correction (ICE) process is invoked. The second phase of ICE begins by iteratively improving the clustering based on the cluster consensus 124 and the quality values 118 (block 206). During this process, the read sequences 116 are automatically "reassigned" from one cluster to another or designated as "orphans" and used to generate new clusters if the sequence reads are determined to not belong to any of the existing clusters, and highly similar clusters are merged, as explained below.

Figure 2 shows further details of the process for iteratively improving the clustering (block 206). The ICE component 112 may begin the iteration process by calculating a probability of each sequence read (S) belonging to each cluster (C) using the quality values (QVs) (block 206-1). This may be accomplished by aligning each of the sequence reads 116 in each of the clusters 122 to each of the cluster consensus C. More specifically, each read Si is aligned to each cluster consensus Cu, where "i" = 1 to the total number of sequence reads, and "u" = 1 to the total number of cluster consensus (in the example shown in Figure 7, i = 12 and u = 4).

If a current sequence read (S) does not align to any of the cluster consensus (C) with a sufficiently high percent of similarity using the process of detecting isoform alignment differences described above (i.e., no isoform hit), then the sequence read is ignored for having a bad probability. In one embodiment, a linear-time algorithm may be used to filter out alignments that have large indels. (See, e.g., Algorithms tor locating extremely conserved elements in multiple sequence alignments. Tseng & Tompa, BMC Bioinformatics, 2009).

If the current sequence read does align to one or more of the cluster consensus, the ICE component 112 calculates a probability of the current read belonging to each of the clusters given the cluster consensus and the QV for the current read:

Pr (Si | Cu, QVs(Si))

If the QVs are unavailable, then:

$$Pr\ (Si\ |\ Cu\ QVs(Si))\ =\ (\theta match)count(match)\ (1/3\ \theta sub)count(sub)\ (1/3\ \theta ins)count(ins)\ (1/3\ \theta del)count(del),$$

where $\theta$ is the probability of a match of a substitution (sub), an insertion (ins), and a deletion (del), respectively.

Referring to Figure 7 as an example, when the probabilities are calculated for read 6, the ICE component 112 determines that the probability of read 6 belonging to cluster consensus C3 is greater than the probability of read 6 (S6) belonging to cluster consensus C4:

Pr (S6 | C3) > Pr (S6 | C4)

This is likely to happen because cluster C3 contains isoforms from the same group as S6.

The output of the probability calculation is a list of probabilities calculated for each of the read sequences belonging to each of the clusters 122. In one embodiment, number of probabilities calculated the total number of nodes/sequence reads multiplied by the total number of clusters, with some probabilities having a value of "unknown".

Referring again to Figure 2, after the probabilities are calculated, the ICE component 112 reassigns individual sequence reads from one cluster to another cluster having a highest calculated probability (block 206-2).

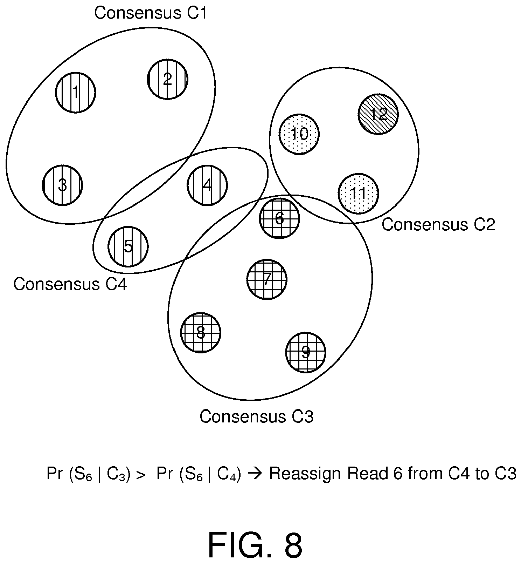

Figure 8 is a diagram illustrating reassignment of a sequence read from one cluster to the cluster having the highest computed probability of membership. This example shows reassignment of read 6 from cluster C4 to C3.

It should be understood that if it is determined that the cluster having the highest calculated probability for the current sequence read is the cluster to which the sequence read is already a member, then no reassignment occurs.

Referring again to Figure 2, according to another aspect of the exemplary embodiment, if either no alignment exists (i.e., the probabilities are unknown) between any of sequence reads and the clusters) or if the linear-time algorithm rejects all alignments for any of the sequence reads, the sequence reads may be considered orphans, and new clusters may then be formed from the orphans (block 206-3). The new clusters may be formed from the orphans using the same procedure as in the initial phase described above.

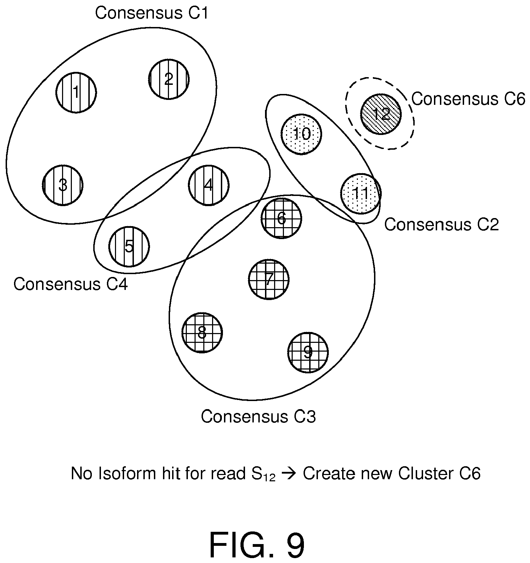

Figure 9 is a diagram illustrating an example of creating a new cluster from an orphan. In this example, it is determined that read S12 does not have an isoform hit. Read S12 is then designated as an orphan and a new cluster C6 is created that contains read S12.

There is one minor problem with the above approach, which is when an orphan is assigned to a new cluster, e.g. C6, which only has one sequence read, the read is its own representative. Therefore, the computed probability of a cluster with one sequence read will always be 1, which means no other cluster will have a higher computed probability for the read and the read will not be reassigned to another cluster. Clusters having only one sequence read may be called singletons and have a lack of members to create diversity, resulting in singleton clusters never having a better cluster to which the node can be reassigned even though one may exist.

According to one embodiment, this problem may be solved by randomly generating a probability for each orphan node. That is, a random number generator may be used to generate a number between a predefined range, between 0 and 1, for example (other ranges are possible). If the random probability is smaller than a predefined threshold probability, e.g., 0.30, then the orphan is reassigned to one of the clusters having a non-zero computed probability of membership for the orphan.

Referring again to Figure 2, the clusters are processed to determine if substantially identical clusters exists, and if so, the clusters are merged into a new cluster (block 206-4). Substantially identical clusters may occur during processing due to approximate maximal clique finding and the iterative consensus calling process. In one embodiment, the determination of whether two clusters are substantially identical based on similarity of their cluster consensus sequence may be controlled through user-defined parameters, e.g., percent of similarity => 95%.

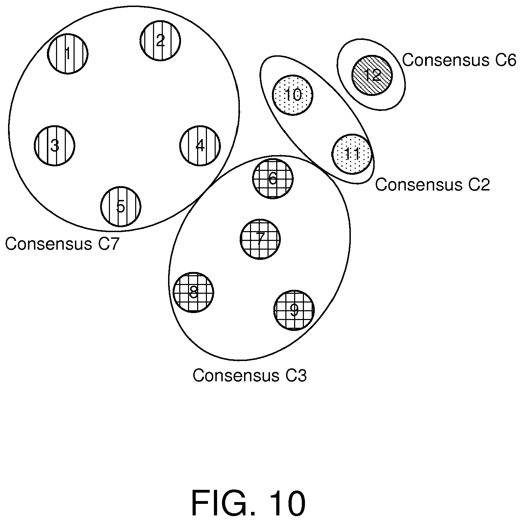

Figure 10 is a diagram illustrating the merger of two clusters. In this example, former cluster C1 and C4 from Figure 9 are determined to be isoform hits and have a threshold percent of similarity greater than 99.5%. Consequently, in Figure 10 clusters C1 and C4 have been merged into a new cluster and a corresponding cluster consensus C7.

Referring again to Figure 2, every time the members of a cluster change, the corresponding cluster consensus may change, as well. Accordingly, the ICE component 112 updates the cluster consensus for each of the changed clusters and updates the probability Pr (Si | Cu, QVs(Si)) for all the sequence reads (block 206-5). This is accomplished by calling the cluster consensus algorithm 111 (block 204) via line 206-6, and thus creating the "iterative loop" of phase II of the iterative clustering for error correction process, the first step of which is recalculating the probabilities of each of the sequence reads (block 206-1).

In one embodiment, the cluster consensus algorithm 111 may generate a cluster consensus 124 for the clusters every time a change happens. However, in one embodiment a predefined threshold may be optionally used for limiting when the cluster consensus algorithm 111 is called based on cluster sizes i.e., if the cluster size is relatively large, calling the cluster consensus algorithm 111 may be skipped in block 206-5 if the number of nodes in a particular cluster is larger than the predefined threshold. In certain embodiments, the cluster consensus algorithm step is parallelizable.

In one embodiment, new additional sequence reads can be added to the existing cluster sets any time during the second phase by aligning the additional sequence reads against all existing consensus sequences. A new sequence is assigned to an existing cluster C if cluster C has the highest posterior probability and the alignment is not rejected. Otherwise, the sequence read is considered an orphan and can form new clusters as in the initial phase described above.

Once the clusters can be improved no further via reassignment of the sequence reads and/or merging the clusters, the iterative clustering for error correction process (block 206) completes.

Once the iterative clustering for error correction process is complete, the polisher component 114 is called to polish the consensus results (block 208). In one embodiment, the polisher component 114 may be based on the Quiver algorithm, as described in U.S. 13/941,442, filed July 12, 2013. As described above, the ICE component 112 calls the cluster consensus algorithm 111 to generate the cluster consensus for each of the clusters. These cluster consensus are used as a "reference" to which the full-length reads 116-1 are aligned during the iterative clustering process in one embodiment.

According to one aspect of exemplary embodiment, the input to the polishing step may comprise the cluster consensus and the non-full-length reads 116-2, which are then aligned to each cluster consensus as a reference. During polishing, the non-full-length reads 116-2 are used to impart full coverage to the sequence data to provide a higher level of consensus using the same "isoform hit" criteria described above. In one embodiment, unlike full-length input sequences, non-full-length reads 116-2 do not have to align exclusively and can belong to multiple clusters. Again, the linear-time algorithm is used to reject non-favorable alignments. Once the non-full-length reads 116-2 are aligned to the cluster consensus, the polisher component 114 generates a final consensus sequence 128 (Figure 1) for each of the clusters. The output from the process may comprise a list of final cluster consensus 128, each of which represents the "consensus" sequence of a cluster. In one embodiment, each cluster may be used to represent a single, unique transcript.

In a further embodiment, the final cluster consensus 128 may be mapped to a genome, wherein redundancy is removed and the isoforms collapsed, thereby generating high-quality, full-length isoforms.

According to the exemplary embodiments, the iterative clustering of sequence reads for error correction process may have many applications. For example, ICE may be used for full-length cDNA sequencing, bioinformatics analysis, and biological applications.

Examples of full-length cDNA sequencing may include, but are not limited to, constructing cDNA libraries enriched in full-length transcripts; size selection using agarose gel or the BluePippin™ system; sequencing transcripts up to 10 kb in full-length; and single-molecule observation of each transcript.

Besides isoform-level clustering to generate high-quality transcript consensus sequences, examples of bioinformatics analysis may include, but are not limited to, identifying putatively full-length transcripts; and detecting artificial chimeras.

Finally, examples of biological applications may include, but are not limited to, novel transcripts; alternative splicing; alternative polyadenylation; retained introns; fusion genes; and anti-sense transcription.

In some embodiments, the system includes a computer-readable medium operatively coupled to the processor that stores instructions for execution by the processor. The instructions may include one or more of the following: instructions for receiving input of sequence reads (and, optionally, reference sequence information),instructions for constructing pre-assembled reads, instructions for aligning sequence reads, instructions for generating string graphs, instructions for generating unitig graphs, instructions for identifying string bundles, instructions for determining primary contigs, instructions for determining associated contigs, instructions for correcting reads, instructions for generating consensus sequences, instructions for generating haplotype sequences, instructions that compute/store information related to various steps of the method (e.g., edges and nodes in a string graph, overlaps and branch points in a string graph, primary and associated contigs, and instructions that record the results of the method.

In certain aspects, the methods are computer-implemented methods. In certain aspects, the algorithm and/or results (e.g., consensus sequences generated) are stored on computer-readable medium, and/or displayed on a screen or on a paper print-out. In certain aspects, the results are further analyzed, e.g., to identify genetic variants, to identify one or more origins of the sequence information, to identify genomic regions conserved between individuals or species, to determine relatedness between two individuals, to provide an individual with a diagnosis or prognosis, or to provide a health care professional with information useful for determining an appropriate therapeutic strategy for a patient.

Furthermore, the functional aspects of the invention that are implemented on a computer or other logic processing systems or circuits, as will be understood to one of ordinary skill in the art, may be implemented or accomplished using any appropriate implementation environment or programming language, such as C, C++, Cobol, Pascal, Java, Java-script, HTML, XML, dHTML, assembly or machine code programming, RTL, etc.

In certain embodiments, the computer-readable media may comprise any combination of a hard drive, auxiliary memory, external memory, server, database, portable memory device (CD-R, DVD, ZIP disk, flash memory cards, etc.), and the like.

In some aspects, the invention includes an article of manufacture for string graph assembly of polyploid genomes that includes a machine-readable medium containing one or more programs which when executed implement the steps of the invention as described herein.

It is to be understood that the above description is intended to be illustrative and not restrictive. It readily should be apparent to one skilled in the art that various modifications may be made to the invention disclosed in this application. The scope of the invention should, therefore, be determined not with reference to the above description, but should instead be determined with reference to the appended claims, along with the full scope of equivalents to which such claims are entitled. Throughout the disclosure various references, patents, patent applications, and publications are cited. All publications mentioned herein are cited for the purpose of describing and disclosing reagents, methodologies and concepts that may be used in connection with the present invention. Nothing herein is to be construed as an admission that these references are prior art in relation to the inventions described herein.

## Claims

1. A method for iterative clustering of sequence reads for error correction, the method performed by at least one software component executing on at least one processor, comprising:

   (A) receiving a set of sequence reads and associated quality values, wherein the associated quality values are estimations of per-position base call accuracy;
   (B) grouping the sequence reads into a set of initial clusters based on sequence similarity;
   (C) generating a cluster consensus for each of the initial clusters;
   (D) iteratively improving the clustering based on the cluster consensus and the quality values associated with the sequence reads, wherein iteratively improving the clustering comprises:

      (i) calculating a probability of each sequence read belonging to each cluster using the quality values,
      (ii) reassigning individual sequence reads from one cluster to another cluster having a highest calculated probability, and
      (iii) merging highly similar clusters; and

   (E) generating and outputting a final cluster consensus for each of the clusters.

2. The method of claim 1, wherein calculating a probability of each sequence read belonging to each cluster using the quality values, further comprises:

   aligning each of the sequence reads in each of the clusters to each of the cluster consensuses;
   responsive to a current sequence read not aligning to any of the cluster consensuses with a sufficiently high percent of similarity, ignoring the sequence read for having a bad probability;
   responsive to the current sequence read (S) aligning to one or more of the cluster consensuses (C), calculating a probability of the current read belonging to each of the clusters given the cluster consensus and the quality values (QVs) for the current read:
   $Pr(S_i \mid C_u, QVs(S_i))$.

3. The method of claim 2, wherein responsive to the QVs being unavailable, then calculating:

$$Pr(S_i \mid C_u \ QVs(S_i)) = (\theta match)count(match) \ (1/3 \ \theta sub)count(sub) \ (1/3 \ \theta ins)count(ins)$$
$$(1/3 \ \theta del)count(del),$$

   wherein $\theta$ is the probability of a match of a substitution (sub), an insertion (ins), and a deletion (del), respectively.

4. The method of claim 1, further comprising:
   responsive to no alignment existing between any of the sequence reads and any of the clusters, considering the sequence reads orphans, and forming new clusters from the orphans, optionally wherein the method further comprises:

   responsive to a new cluster having only one sequence read, randomly generating a random probability for each orphan node; and
   responsive to random probability being smaller than a predefined threshold probability, reassigning the orphan to one of the clusters having a non-zero computed probability of membership for the orphan.

5. The method of claim 1, wherein the sequence reads received comprise full-length long reads, and wherein the generating and outputting the final cluster consensus further comprises:
inputting non-full-length reads into a final polishing processes that generates the final cluster consensus, optionally wherein the reads comprise full-length long reads range in length from 0.5 kb to 1, 2, 3, 5, 10, 15, or 20 kb.

6. The method of claim 1, wherein grouping the sequence reads into a set of initial clusters based on sequence similarity further comprises:

aligning the sequence reads to create aligned reads;
building similarity graphs using the aligned reads; and
finding maximal cliques using the similarity graphs, optionally wherein finding maximal cliques comprises: non-deterministically partitioning the similarity graphs into non-overlapping maximal cliques.

7. An executable software product stored on a computer-readable medium containing program instructions for iterative clustering of sequence reads for error correction, the program instructions executing on at least one processor, wherein the executable software product is for carrying out a method of any one of claims 1 to 6.

8. A system for iterative clustering of sequence reads for error correction, comprising:

a memory; and
a processor coupled to the memory configured to:

(A) receive a set of sequence reads and associated quality values, wherein the associated quality values are estimations of per-position base call accuracy;
(B) group the sequence reads into a set of initial clusters based on sequence similarity;
(C) generate a cluster consensus for each of the initial clusters;
(D) iteratively improve the clustering based on the cluster consensus and the quality values associated with the sequence reads, by a method comprising:

(i) calculating a probability of each sequence read belonging to each cluster using the quality values,
(ii) reassigning individual sequence reads from one cluster to another cluster having a highest calculated probability, and
(iii) merging highly similar clusters; and

(F) generate and output a final cluster consensus for each of the clusters.

9. The system of claim 8, wherein calculating a probability of each sequence read belonging to each cluster using the quality values, further comprises:

aligning each of the sequence reads in each of the clusters to each of the cluster consensus;
responsive to a current sequence read not aligning to any of the cluster consensuses with a sufficiently high percent of similarity, ignoring the sequence read for having a bad probability;
responsive to the current sequence read (S) aligning to one or more of the cluster consensuses (C), calculating a probability of the current read belonging to each of the clusters given the cluster consensus and the quality values (QVs) for the current read:
Pr(Si | Cu, QVs(Si)).

10. The system of claim 9, wherein responsive to the QVs being unavailable, then calculating:

$$Pr(Si \mid Cu \ QVs(Si)) = (\theta match)count(match) \ (1/3 \ \theta sub)count(sub) \ (1/3 \ \theta ins)count(ins) \ (1/3 \ \theta del)count(del),$$

wherein $\theta$ is the probability of a match of a substitution (sub), an insertion (ins), and a deletion (del), respectively.

11. The system of claim 9, further comprising:
responsive to no alignment existing between any of the sequence reads and any of the clusters, considering the sequence reads orphans, and forming new clusters from the orphans; optionally wherein the system further comprises:

responsive to a new cluster having only one sequence read, randomly generating a random probability for each orphan node; and
responsive to random probability being smaller than a predefined threshold probability, reassigning the orphan to one of the clusters having a non-zero computed probability of membership for the orphan.

12. The system of claim 8, wherein the sequence reads received comprise full-length long reads, and wherein the generating and outputting the final cluster consensus further comprises:
inputting non-full-length reads into a final polishing processes that generates the final cluster consensus, optionally wherein the reads comprise full-length long reads range in length from 0.5 kb to 1, 2, 3, 5, 10, 15, or 20 kb.

13. The system of claim 8, wherein grouping the sequence reads into a set of initial clusters based on sequence similarity further comprises:

aligning the sequence reads to create aligned reads;
building similarity graphs using the aligned reads; and
finding maximal cliques using the similarity graphs, optionally wherein finding maximal cliques comprises: non-deterministically partitioning the similarity graphs into non-overlapping maximal cliques.

**Patentansprüche**

1. Verfahren zum iterativen Clustern von Sequenzauslesungen zur Fehlerkorrektur, wobei das Verfahren von zumindest einer Softwarekomponente durchgeführt wird, die auf zumindest einem Prozessor ausgeführt wird, das Folgendes umfasst:

(A) Empfangen eines Satzes von Sequenzauslesungen und zugehörigen Qualitätswerten, wobei die zugehörigen Qualitätswerte Schätzungen der Basenfolgenzuordnungsgenauigkeit pro Position sind;
(B) Gruppieren der Sequenzauslesungen in einen Satz von Ausgangsclustern basierend auf der Sequenzähnlichkeit;
(C) Erzeugen eines Cluster-Konsensus für jedes der Ausgangscluster;
(D) iteratives Verbessern des Clusterings basierend auf dem Cluster-Konsensus und den den Sequenzauslesungen zugehörigen Qualitätswerten, wobei das iterative Verbessern des Clusterings Folgendes umfasst:

(i) Berechnen einer Wahrscheinlichkeit, dass jede Sequenzauslesung zu jedem Cluster gehört, unter Verwendung der Qualitätswerte,
(ii) erneutes Zuordnen einzelner Sequenzauslesungen von einem Cluster zu einem anderen Cluster mit der höchsten berechneten Wahrscheinlichkeit und
(iii) Zusammenführen von stark ähnlichen Clustern; und

(E) Erzeugen und Ausgeben eines endgültigen Cluster-Konsensus für jedes der Cluster.

2. Verfahren nach Anspruch 1, wobei das Berechnen einer Wahrscheinlichkeit, dass jede Sequenzauslesung zu jedem Cluster gehört, unter Verwendung der Qualitätswerte weiters Folgendes umfasst:

Alignieren jeder der Sequenzauslesungen in jedem der Cluster an jeder der Cluster-Konsensus;
als Reaktion darauf, dass eine aktuelle Sequenzauslesung, die nicht mit einem ausreichend hohen Ähnlichkeitsprozentanteil an den Cluster-Konsensus aligniert ist, Ignorieren der Sequenzauslesung aufgrund einer schlechten Wahrscheinlichkeit;
als Reaktion darauf, dass die aktuelle Sequenzauslesung (S) an einer oder mehreren der Cluster-Konsensus (C) aligniert ist, Berechnen einer Wahrscheinlichkeit, dass die aktuelle Auslesung zu jedem der Cluster gehört, unter Annahme des Cluster-Konsensus und der Qualitätswerte (QV) für die aktuelle Auslesung:
Pr(Si | Cu, QVs(Si)).

3. Verfahren nach Anspruch 2, wobei als Reaktion darauf, dass die QVs nicht verfügbar sind, Folgendes berechnet wird:

$$Pr(Si \mid Cu \ QVs(Si)) = (\theta match)count(match) \ (1/3 \ \theta sub)count(sub) \ (1/3 \ \theta ins)count(ins)$$

$$(1/3 \ \theta del)count(del),$$

worin θ die Wahrscheinlichkeit eines Konsensus (match) einer Substitution (sub), einer Insertion (ins) bzw. einer Deletion (del) ist.

4. Verfahren nach Anspruch 1, das ferner Folgendes umfasst:
als Reaktion darauf, dass keine Alignierung zwischen keiner der Sequenzauslesungen und keinem der Cluster besteht, Berücksichtigen der verwaisten Sequenzauslesungen und Bilden neuer Cluster aus den verwaisten Auslesungen, wobei das Verfahren gegebenenfalls ferner Folgendes umfasst:

als Reaktion darauf, dass ein neues Cluster nur eine Sequenzauslesung aufweist, zufälliges Erzeugen einer Zufallswahrscheinlichkeit für jeden verwaisten Knoten; und
als Reaktion darauf, dass eine Zufallswahrscheinlichkeit kleiner ist als eine vorbestimmte Schwellenwahrscheinlichkeit, erneutes Zuordnen des verwaisten Knotens zu einem der Cluster mit einer berechneten Wahrscheinlichkeit der Zugehörigkeit für die verwaiste Auslesung, die nicht null ist.

5. Verfahren nach Anspruch 1, wobei die empfangenen Sequenzauslesungen Volllängen-Auslesungen umfassen und wobei das Erzeugen und die Ausgabe des endgültigen Cluster-Konsensus ferner Folgendes umfasst:
Eingeben von Nicht-Volllängen-Auslesungen in einen endgültigen Feinabstimmungsprozess, der die endgültigen Cluster-Konsensus erzeugt, wobei die Auslesungen gegebenenfalls Volllängen-Auslesungen mit einer Länge im Bereich von 0,5 kb bis 1, 2, 3, 5, 10, 15 oder 20 kb umfassen.

6. Verfahren nach Anspruch 1, wobei das Gruppieren der Sequenzauslesungen in einen Satz von Ausgangsclustern basierend auf der Sequenzähnlichkeit ferner Folgendes umfasst:

Alignieren der Sequenzauslesungen, um alignierte Auslesungen zu erzeugen;
Erstellen von Ähnlichkeitskurven unter Verwendung der alignierten Auslesungen; und
Finden von maximalen Cliquen unter Verwendung der Ähnlichkeitskurven, wobei gegebenenfalls das Finden der maximalen Cliquen Folgendes umfasst: nichtdeterministisches Partitionieren der Ähnlichkeitskurven in nichtüberlagernde maximale Cliquen.

7. Ausführbares Softwareprodukt, das auf einem computerlesbaren Medium gespeichert ist, enthaltend Programmbefehle zum iterativen Clustern von Sequenzauslesungen zur Fehlerkorrektur, wobei die Programmbefehle auf zumindest einem Prozessor ausgeführt werden, wobei das ausführbare Softwareprodukt zum Durchführen eines Verfahrens nach einem der Ansprüche 1 bis 6 dient.

8. System zum iterativen Clustern von Sequenzauslesungen zur Fehlerkorrektur, das Folgendes umfasst:

einen Arbeitsspeicher; und
einen Prozessor, der mit dem Arbeitsspeicher gekoppelt und konfiguriert ist:

(A) einen Satz aus Sequenzauslesungen und zugehörigen Qualitätswerten zu empfangen, wobei die zugehörigen Qualitätswerte Schätzungen der Basenfolgenzuordnungsgenauigkeit pro Position sind;
(B) die Sequenzauslesungen basierend auf der Sequenzähnlichkeit in einen Satz von Ausgangsclustern zu gruppieren;
(C) einen Cluster-Konsensus für jedes der Ausgangscluster zu erzeugen;
(D) das Clustering basierend auf dem Cluster-Konsensus und den Qualitätswerten, die den Sequenzauslesungen zugehören, iterativ über ein Verfahren zu verbessern, das Folgendes umfasst:

(i) Berechnen einer Wahrscheinlichkeit, dass jede Sequenzauslesung zu jedem Cluster gehört, unter Verwendung der Qualitätswerte,
(ii) erneutes Zuordnen einzelner Sequenzauslesungen von einem Cluster zu einem anderen Cluster mit der höchsten berechneten Wahrscheinlichkeit und
(iii) Zusammenführen von stark ähnlichen Clustern; und

(E) Erzeugen und Ausgeben eines endgültigen Cluster-Konsensus für jedes der Cluster.

9. System nach Anspruch 8, wobei das Berechnen einer Wahrscheinlichkeit, dass jede Sequenzauslesung zu jedem Cluster gehört, unter Verwendung der Qualitätswerte ferner Folgendes umfasst:

Alignieren jeder der Sequenzauslesungen in jedem der Cluster mit jedem der Cluster-Konsensus;
als Reaktion darauf, dass eine aktuelle Sequenzauslesung, die nicht mit einem ausreichend hohen Ähnlichkeitsprozentanteil an den Cluster-Konsensus aligniert ist, Ignorieren der Sequenzauslesung aufgrund einer schlechten Wahrscheinlichkeit;
als Reaktion darauf, dass die aktuelle Sequenzauslesung (S) an einem oder mehreren der Cluster-Konsensus (C) aligniert ist, Berechnen einer Wahrscheinlichkeit, dass die aktuelle Auslesung zu jedem der Cluster gehört, unter Annahme der Cluster-Konesnsus und der Qualitätswerte (QV) für die aktuelle Auslesung:
Pr(Si | Cu, QVs(Si)).

10. System nach Anspruch 9, wobei als Reaktion darauf, dass die QVs nicht verfügbar sind, dann Folgendes berechnet wird:

$$Pr(Si | Cu \ QVs(Si)) = (\theta match)count(match) \ (1/3 \ \theta sub)count(sub) \ (1/3 \ \theta ins)count(ins) \ (1/3 \ \theta del)count(del),$$

worin θ die Wahrscheinlichkeit eines Konsensus (match) einer Substitution (sub), einer Insertion (ins) bzw. einer Deletion (del) ist.

11. System nach Anspruch 9, das ferner Folgendes umfasst:
als Reaktion darauf, dass keine Alignierung zwischen keiner der Sequenzauslesungen und keinem der Cluster besteht, Berücksichtigen der verwaisten Sequenzauslesungen und Bilden neuer Cluster aus den verwaisten Auslesungen, wobei das Verfahren gegebenenfalls ferner Folgendes umfasst:

als Reaktion darauf, dass ein neues Cluster nur eine Sequenzauslesung aufweist, zufälliges Erzeugen einer Zufallswahrscheinlichkeit für jeden verwaisten Knoten; und
als Reaktion darauf, dass eine Zufallswahrscheinlichkeit kleiner ist als eine vorbestimmte Schwellenwahrscheinlichkeit, erneutes Zuordnen des verwaisten Knotens zu einem der Cluster mit einer berechneten Wahrscheinlichkeit der Zugehörigkeit für die verwaiste Auslesung, die nicht null ist.

12. System nach Anspruch 8, wobei die empfangenen Sequenzauslesungen Volllängen-Auslesungen umfassen und wobei das Erzeugen und die Ausgabe des endgültigen Cluster-Konsensus ferner Folgendes umfasst:
Eingeben von Nicht-Volllängen-Auslesungen in einen endgültigen Feinabstimmungsprozess, der den endgültigen Cluster-Konsensus erzeugt, wobei die Auslesungen gegebenenfalls Volllängen-Auslesungen mit einer Länge im Bereich von 0,5 kb bis 1, 2, 3, 5, 10, 15 oder 20 kb umfassen.

13. System nach Anspruch 8, wobei das Gruppieren der Sequenzauslesungen in einen Satz von Ausgangsclustern basierend auf der Sequenzähnlichkeit ferner Folgendes umfasst:

Alignieren der Sequenzauslesungen, um alignierte Auslesungen zu erzeugen;
Erstellen von Ähnlichkeitskurven unter Verwendung der alignierten Auslesungen; und
Finden von maximalen Cliquen unter Verwendung der Ähnlichkeitskurven, wobei gegebenenfalls das Finden der maximalen Cliquen Folgendes umfasst: nichtdeterministisches Partitionieren der Ähnlichkeitskurven in nichtüberlagernde maximale Cliquen.

**Revendications**

1. Procédé de regroupement itératif de lectures de séquences en vue d'une correction d'erreur, le procédé étant mis en œuvre par au moins un composant logiciel s'exécutant sur au moins un processeur, comprenant les étapes ci-dessous consistant à :

(A) recevoir un ensemble de lectures de séquences et des valeurs de qualité associées, dans lequel les valeurs de qualité associées correspondent à des estimations de précision d'appels sur une base « par position » ;
(B) grouper les lectures de séquences en un ensemble de groupes initiaux sur la base d'une similarité de séquences ;

(C) générer un consensus de groupe pour chacun des groupes initiaux ;

(D) améliorer itérativement le regroupement sur la base du consensus de groupe et des valeurs de qualité associées aux lectures de séquences, dans lequel l'étape d'amélioration itérative du regroupement comprend les étapes ci-dessous consistant à :

(i) calculer une probabilité que chaque lecture de séquence appartienne à chaque groupe, en utilisant les valeurs de qualité ;

(ii) réaffecter des lectures de séquences individuelles d'un groupe à un autre groupe présentant une probabilité calculée la plus élevée ; et

(iii) fusionner des groupes hautement similaires ; et

(E) générer et fournir en sortie un consensus de groupe final pour chacun des groupes.

2. Procédé selon la revendication 1, dans lequel l'étape consistant à calculer une probabilité que chaque lecture de séquence appartienne à chaque groupe en utilisant les valeurs de qualité, comprend en outre les étapes ci-dessous consistant à :

aligner chacune des lectures de séquences dans chacun des groupes sur chacun des consensus de groupe ;

en réponse au fait qu'une lecture de séquence en cours ne s'aligne sur aucun des consensus de groupe avec un pourcentage de similarité suffisamment élevé, ignorer la lecture de séquence en raison de sa mauvaise probabilité ;

en réponse au fait que la lecture de séquence en cours (S) s'aligne sur un ou plusieurs des consensus de groupe (C), calculer une probabilité que la lecture de séquence en cours appartienne à chacun des groupes étant donné le consensus de groupe et les valeurs de qualité (QV) pour la lecture de séquence en cours : Pr (Si | Cu, QVs(Si)).

3. Procédé selon la revendication 2, dans lequel, en réponse à l'indisponibilité des valeurs de qualité, QV, le procédé comprend l'étape consistant à calculer ensuite :

$$\text{Pr (Si | Cu, QVs(Si))} = (\theta\text{match})\text{count(match)} \quad (1/3\ \theta\text{sub})\text{count(sub)} \quad (1/3\ \theta\text{ins})\text{count(ins)} \quad (1/3\ \theta\text{del})\text{count(del)},$$

dans lequel « θ » est la probabilité d'une correspondance d'une substitution (sub), d'une insertion (ins), et d'une suppression (del), respectivement.

4. Procédé selon la revendication 1, comprenant en outre les étapes ci-dessous consistant à :

en réponse à l'absence d'alignement existant entre l'une quelconque des lectures de séquences et l'un quelconque des groupes, considérer les lectures de séquences comme des orphelins, et former de nouveaux groupes à partir des orphelins, dans lequel le procédé comprend en outre, facultativement, les étapes ci-dessous consistant à :

en réponse au fait qu'un nouveau groupe ne présente qu'une seule lecture de séquence, générer de manière aléatoire une probabilité aléatoire pour chaque nœud orphelin ; et

en réponse au fait qu'une probabilité aléatoire est inférieure à une probabilité de seuil prédéfinie, réaffecter l'orphelin à l'un des groupes présentant une probabilité calculée non nulle d'appartenance pour l'orphelin.

5. Procédé selon la revendication 1, dans lequel les lectures de séquences reçues comprennent des lectures longues complètes, et dans lequel l'étape de génération et de fourniture en sortie du consensus de groupe final comprend en outre l'étape ci-dessous consistant à :

appliquer en entrée des lectures non complètes dans le cadre d'un processus de polissage final qui génère le consensus de groupe final, facultativement dans lequel les lectures comprennent des lectures longues complètes dont la longueur varie de 0,5 kb à 1, 2, 3, 5, 10, 15 ou 20 kb.

6. Procédé selon la revendication 1, dans lequel l'étape de groupement des lectures de séquences en un ensemble de groupes initiaux sur la base d'une similarité de séquences comprend en outre les étapes ci-dessous consistant à :

aligner les lectures de séquences en vue de créer des lectures alignées ;
construire des graphes de similarité en utilisant les lectures alignées ; et
rechercher des cliques maximales en utilisant les graphes de similarité, facultativement dans lequel l'étape de recherche de cliques maximales comprend l'étape ci-après consistant à : partitionner de manière non déterministe les graphes de similarité en des cliques maximales sans chevauchement.

7. Produit logiciel exécutable stocké sur un support lisible par ordinateur contenant des instructions de programme en vue d'un regroupement itératif de lectures de séquences pour une correction d'erreur, les instructions de programme s'exécutant sur au moins un processeur, dans lequel le produit logiciel exécutable est destiné à mettre en œuvre un procédé selon l'une quelconque des revendications 1 à 6.

8. Système pour un regroupement itératif de lectures de séquences pour une correction d'erreur, comprenant :

une mémoire ; et
un processeur couplé à la mémoire, configuré de manière à :

(A) recevoir un ensemble de lectures de séquences et des valeurs de qualité associées, dans lequel les valeurs de qualité associées sont des estimations de précision d'appels sur une base « par position » ;
(B) grouper les lectures de séquences en un ensemble de groupes initiaux sur la base d'une similarité de séquences ;
(C) générer un consensus de groupe pour chacun des groupes initiaux ;
(D) améliorer itérativement le regroupement sur la base du consensus de groupe et des valeurs de qualité associées aux lectures de séquences, en mettant en œuvre un procédé comprenant les étapes ci-dessous consistant à :

(i) calculer une probabilité que chaque lecture de séquence appartienne à chaque groupe, en utilisant les valeurs de qualité ;
(ii) réaffecter des lectures de séquences individuelles d'un groupe à un autre groupe présentant une probabilité calculée la plus élevée ; et
(iii) fusionner des groupes hautement similaires ; et

(E) générer et fournir en sortie un consensus de groupe final pour chacun des groupes.

9. Système selon la revendication 8, dans lequel l'étape consistant à calculer une probabilité que chaque lecture de séquence appartienne à chaque groupe en utilisant les valeurs de qualité, comprend en outre les étapes ci-dessous consistant à :

aligner chacune des lectures de séquences dans chacun des groupes sur chacun des consensus de groupe ;
en réponse au fait qu'une lecture de séquence en cours ne s'aligne sur aucun des consensus de groupe avec un pourcentage de similarité suffisamment élevé, ignorer la lecture de séquence en raison de sa mauvaise probabilité ;
en réponse au fait que la lecture de séquence en cours (S) s'aligne sur un ou plusieurs des consensus de groupe (C), calculer une probabilité que la lecture de séquence en cours appartienne à chacun des groupes étant donné le consensus de groupe et les valeurs de qualité (QV) pour la lecture de séquence en cours :
Pr (Si | Cu, QVs(Si)).

10. Système selon la revendication 9, dans lequel, en réponse à l'indisponibilité des valeurs de qualité, QV, calculer ensuite :

$$Pr(Si \mid Cu, QVs(Si)) = (\theta match)count(match) \quad (1/3\ \theta sub)count(sub) \quad (1/3\ \theta ins)count(ins) \quad (1/3\ \theta del)count(del),$$

dans lequel « $\theta$ » est la probabilité d'une correspondance d'une substitution (sub), d'une insertion (ins), et d'une suppression (del), respectivement.

11. Système selon la revendication 9, comprenant en outre les étapes ci-dessous consistant à :

en réponse à l'absence d'alignement existant entre l'une quelconque des lectures de séquences et l'un quelconque des groupes, considérer les lectures de séquences comme des orphelins, et former de nouveaux groupes à partir des orphelins, dans lequel le système comprend en outre, facultativement, les étapes ci-dessous consistant à :

en réponse au fait qu'un nouveau groupe ne présente qu'une seule lecture de séquence, générer de manière aléatoire une probabilité aléatoire pour chaque nœud orphelin ; et
en réponse au fait qu'une probabilité aléatoire est inférieure à une probabilité de seuil prédéfinie, réaffecter l'orphelin à l'un des groupes présentant une probabilité calculée non nulle d'appartenance pour l'orphelin.

12. Système selon la revendication 8, dans lequel les lectures de séquences reçues comprennent des lectures longues complètes, et dans lequel l'étape de génération et de fourniture en sortie du consensus de groupe final comprend en outre l'étape ci-dessous consistant à :
appliquer en entrée des lectures non complètes dans le cadre d'un processus de polissage final qui génère le consensus de groupe final, facultativement dans lequel les lectures comprennent des lectures longues complètes dont la longueur varie de 0,5 kb à 1, 2, 3, 5, 10, 15 ou 20 kb.

13. Système selon la revendication 8, dans lequel l'étape de groupement des lectures de séquences en un ensemble de groupes initiaux sur la base d'une similarité de séquences comprend en outre les étapes ci-dessous consistant à :

aligner les lectures de séquences en vue de créer des lectures alignées ;
construire des graphes de similarité en utilisant les lectures alignées ; et
rechercher des cliques maximales en utilisant les graphes de similarité, facultativement dans lequel l'étape de recherche de cliques maximales comprend l'étape ci-après consistant à : partitionner de manière non déterministe les graphes de similarité en des cliques maximales sans chevauchement.

FIG. 1

Phase I

Receive a set of sequence reads and
associated quality values (QVs)
200

Group the sequence reads into a set of
initial clusters based on sequence
similarity
202

Align sequence reads
202-1

Build Similarity Graphs
202-2

Find Maximal Cliques
202-3

Generate an interim cluster consensus
for each of the initial clusters
204

Calculate Prob of each sequence (s)
belonging to each cluster (c) using
quality values: Pr $(S_i \mid C_u, QVs(S_i))$
206-1

Reassign individual sequence reads
from one cluster to another cluster
having the highest calculated probability
206-2

Phase II

Iteratively improve the clustering based
on the cluster consensus and the quality
values
206

If no alignment exists between any of
the sequence reads and the clusters,
consider the sequence reads orphans
and form new clusters from the orphans
206-3

Generate and output a final cluster
consensus for each of the clusters
208

Merge clusters having substantially
similar consensus
206-4

206-6

Update cluster consensus for each
changed cluster and update
Pr $(S_i \mid C_u, QVs(S_i))$ for all reads
206-5

FIG. 2

```
nMatch: 1668
nMisMatch: 1
    nIns: 2
    nDel: 11
    %sim: 99.1677
    Score: -8269                                           raw 9,230 bp, 6 passes
   ┌─  Query: m130517_074204_sherri_c100509232550000001823074508221393_s1_p0/2382/1739_58_CCS/0_1675
   └─  Target: m130517_144550_sherri_c100509232550000001823074508221396_s1_p0/71648/1742_57_CCS
       Model: a hybrid of global/local non-affine alignment
                                                          raw 13,863 bp, 8 passes
    Raw score: -8269
 QueryRange: 4 -> 1675 of 1675
TargetRange: 0 -> 1680 of 1680
   4   AGGGCGGGGAGGTGGGCAAGATGGCGCTTG-CGAGTGATTCTCCTCGAAT
       |||||||||||||||||||||||||||||*||||||||||||||||||||
   0   AGGGCGGGGAGGTGGGCAAGATGGCGCTTGCCGAGTGATTCTCCTCGAAT

  53   ACCTCCTGCCGGCGCGGAGACACCGGGGC-GGGGGTCCTGCCGCAACTAC
       |||||||||||||||||||||||||||||*||||||||||||||||||||
  50   ACCTCCTGCCGGCGCGGAGACACCGGGGCGGGGGTCCTGCCGCAACTAC

 102   CTCCCTTCCTCCTCTCCCCGC-CCCCGGAGCCTTCATCCTTCCCTT-CCC
       ||||||||||||||||||||*|*|||||||||||||||||||||||*|||
 100   CTCCCTTCCTCCTCTCCCC-CGCCCCGGAGCCTTCATCCTTCCCTTCCCC

 150   CCCCACCTCGAGGGGCGGGCCTGGTTCCC-GGACA-CATGTCGGACTCTG
       ||||||||||||||||||||||||||||*|||||*|||||||||||||||
 149   CCCCACCTCGAGGGGCGGGCCTGGTTCCCGGGACACCATGTCGGACTCTG

 198   AGGAGGAGAGCCAGGACCGGCAACTGAAAATCGTCGTGCT-GGGGACGGC
       |||||||||||||||||||||||||||||||||||||||*||||||||||
 199   AGGAGGAGAGCCAGGACCGGCAACTGAAAATCGTCGTGCTGGGGGACGGC

       |
       |
       |
       !
```

Aligned Reads 300

FIG. 3

Similarity
Graphs
400

402

404

FIG. 4

```
S               +     +
I                                    +
D                         +
745  GAACAGT~~CAGCGTATTGTCAGGGGCAGAAATAGTGAAGGAC~~~~~~~AGAAAAA     Aligned
     ||||||||**|||||||||||||||||||||||||**||*||****** ** |||||||   ─ Reads
746  GAACAGTCACAGCGTATTGTCAGGGGCAGAAATAGT~~AGTACCAAAAAAAGAAAAA        300
S        +
I                                              +++++++
D                    +
```

0000000110000000000000000000000000000110010000000010000000

Difference Array
500

# FIG. 5

FIG. 6

FIG. 7

Consensus C1

Consensus C2

Consensus C4

Consensus C3

$Pr\,(S_6 \mid C_3) > Pr\,(S_6 \mid C_4) \rightarrow$ Reassign Read 6 from C4 to C3

# FIG. 8

Consensus C1

Consensus C6

Consensus C2

Consensus C4

Consensus C3

No Isoform hit for read $S_{12}$ → Create new Cluster C6

FIG. 9

Consensus C6

Consensus C2

Consensus C7

Consensus C3

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 94144213
- US 20120330566 A

### Non-patent literature cited in the description

- **R. B.** Ash. Real Analysis and Probability. Academic Press, 1972
- **D. T. BERTSEKAS ; J. N. TSITSIKLIS.** Introduction to Probability. 2002
- **K. L. CHUNG.** Markov Chains with Stationary Transition Probabilities. 1967
- **W. B. DAVENPORT ; W. L ROOT.** An Introduction to the Theory of Random Signals and Noise. McGraw-Hill, 1958
- **S. M. KAY.** *Fundamentals of Statistical Processing,* 1998, vol. 1-2
- **MONSOON H. HAYES.** *Statistical Digital Signal Processing and Modeling,* 1996
- **R.M. GRAY ; L.D. DAVISSON.** *Introduction to Statistical Signal Processing*
- Modern Spectral Estimation: Theory and Application/Book and Disk. **STEVEN M. KAY.** Signal Processing Series. Prentice-Hall, January 1988
- **STEVEN M. KAY.** *Modern Spectral Estimation: Theory and Application,* March 1999
- **BURKHARD BUTTKUS.** Spectral Analysis and Filter Theory in Applied Geophysics. 11 May 2000
- **DONALD B. PERCIVAL ; ANDREW T. WALDEN.** Spectral Analysis for Physical Applications. 25 June 1993
- **J. L. STARCK ; F. MURTAGH.** Astronomical Image and Data Analysis. *Astronomy and Astrophysics Library,* 25 September 2006
- **DANIEL S. SEM.** *Spectral Techniques In Proteomics,* 30 March 2007
- **DHAMMIKA AMARATUNGA ; JAVIER CABRERA.** Exploration and Analysis of DNA Microarray and Protein Array Data. *Wiley Series in Probability and Statistics,* 21 October 2003
- **ZOGARDI et al.** Error correction of next-generation sequencing data and reliable estimation of HIV quasispecies. *Nucl. Acids. Res.,* 2010
- **ABELLO et al.** On maximum clique problems in very large graphs. *AT&T labs Research Technical Report,* 1998
- **TSENG ; TOMPA.** Algorithms for Locating Extremely Conserved Elements in Multiple Sequence Alignments. *BMC Bioinformatics,* 2009
- **CHIN et al.** Nonhybrid, finished microbial genome assemblies from long-read SMRT sequencing data. *Nature Methods,* 2013